# FASCICULE DE BREVET EUROPEEN

(11) **EP 2 083 837 B1**
(45) Date de publication et mention de la délivrance du brevet: **07.01.2015**
(21) Numéro de dépôt: 07866446.3
(22) Date de dépôt: 26.10.2007
(51) Int. Cl.: A61K 35/74, A61P 25/04, A61P 35/00

(54) **UTILISATION THERAPEUTIQUE D'AU MOINS UNE NEUROTOXINE BOTULIQUE DANS LE TRAITEMENT DE LA DOULEUR INDUITE PAR AU MOINS UN AGENT ANTI-CANCEREUX**
VERWENDUNG VON BOTULINUM NEUROTOXIN ZUR BEHANDLUNG VON ANTIIKREBSMITTEL- INDUZIERTEM SCHMERZ
THERAPEUTIC USE OF AT LEAST ONE BOTULIC NEUROTOXIN IN THE TREATMENT OF PAIN INDUCED BY AT LEAST ONE ANTINEOPLASTIC AGENT

(30) Priorité: 27.10.2006 FR 0609435
(43) Date de publication de la demande: 05.08.2009
(73) Titulaire: IPSEN PHARMA S.A.S., 92100 Boulogne-Billancourt (FR)
(72) Inventeur: FAVRE, Christine, 91530 Saint Maurice Montcouronne (FR); AUGUET, Michel, 91120 Palaiseau (FR); CHABRIER DE LASSAUNIERE, Pierre-Etienne, 75016 Paris (FR)
(74) Mandataire: Retzler, Charlotte
(86) Numéro de dépôt international: PCT/FR2007/001773
(87) Numéro de publication internationale: WO 2008/059126

(56) Documents cités:
- WO-A-01/26736
- WO-A-01/78760
- WO-A-95/17904
- US-A1- 2002 192 239
- DATABASE WPI [Online] DERWENT PUBLICATIONS LTD., LONDON, GB; DW: 200377 XP002439047 Database accession no. AN:2003-826007
- Judith A Paice: "Mechanisms and management of neuropathic pain in cancer", The journal of supportive oncology, 1 July 2003 (2003-07-01), page 107, XP055130600, United States Retrieved from the Internet: URL:http://www.ncbi.nlm.nih.gov/pubmed/153 52654
- JUAN P. CATA ET AL: "The effects of thalidomide and minocycline on taxol-induced hyperalgesia in rats", BRAIN RESEARCH, vol. 1229, 1 September 2008 (2008-09-01), pages 100-110, XP055130597, ISSN: 0006-8993, DOI: 10.1016/j.brainres.2008.07.001
- CUI MINGLEI ET AL: "Subcutaneous administration of botulinum toxin A reduces formalin-induced pain", PAIN, ELSEVIER SCIENCE PUBLISHERS, AMSTERDAM, NL, vol. 107, no. 1-2, 1 January 2004 (2004-01-01), pages 125-133, XP002337053, ISSN: 0304-3959, DOI: 10.1016/J.PAIN.2003.10.008
- ROSEMARY C POLOMANO ET AL: "A painful peripheral neuropathy in the rat produced by the chemotherapeutic drug, paclitaxel", PAIN, vol. 94, no. 3, 1 December 2001 (2001-12-01), pages 293-304, XP055130591, ISSN: 0304-3959, DOI: 10.1016/S0304-3959(01)00363-3

## Description

La présente invention a pour objet l'utilisation d'au moins une neurotoxine botulique pour l'obtention d'un médicament destiné à traiter ou prévenir la ou les douleurs post-chimiothérapiques associées à un traitement anti-cancéreux.

La neuropathie post-chimiothérapique qui est une douleur provoquée par un traitement chimique pour lutter contre le cancer, reste encore aujourd'hui une pathologie difficile à soulager ou à guérir. Ce type de douleur est à distinguer de la douleur provoquée par les tumeurs cancéreuses elles-mêmes. En effet la neuropathie post-chimiothérapique est induite par l'agent anti-cancéreux administré aux patients aux fins de les soigner.

Les douleurs neuropathiques post-chimiothérapiques ont des caractéristiques sémiologiques particulières. Généralement ces douleurs se caractérisent entre-autres par une douleur continue, diffuse, sans rythme mécanique ou inflammatoire, du type brûlure. Sur ce fond douloureux continu peuvent survenir d'autres symptômes : des accès spontanés du type élancements, picotements plus particulièrement des picotements au niveau des extrémités des membres, ou encore des décharges électriques. La topographie de ces symptômes correspond à une distribution compatible avec une systématisation périphérique ou centrale. En d'autres termes la topographie de ces douleurs neuropathiques post-chimiothérapiques est indépendante de la topographie des tumeurs cancéreuses.

Parmi les traitements connus de cette douleur, on peut citer par exemple l'administration d'anticonvulsivants, d'anti-dépresseurs ou de composés opiacés tels que la morphine, qui a été isolée au tout début du XIX^{e} siècle par un pharmacien allemand, Friedrich Sertürner, à partir de l'opium dont elle est le constituant principal.

Par ailleurs l'Organisation Mondiale de la Santé préconise trois paliers de prescription des médicaments antalgiques, règle qui se révèle efficace chez 70 % des patients :
Palier I : antalgiques non opioides pour des douleurs faibles à modérées
Palier II : antalgiques opioides faibles associés aux non opioides pour des douleurs modérées à intenses.
Palier III : antalgiques opioides forts pour des douleurs intenses à très intenses.

Cependant l'usage des composés actuellement disponibles qui permettent de réduire la douleur provoquée par un traitement anti-cancéreux n'est pas satisfaisant car il nécessite l'emploi de doses élevées de composés, ou bien une ré-administration fréquente du composé avec un possible développement d'une résistance au composé ou accoutumance. De plus ces traitements anti-douleurs peuvent provoquer des effets secondaires, qui s'ajoutent à ceux déjà provoqués par le cancer.

Il est donc devenu nécessaire de trouver un autre moyen pour traiter ces douleurs neuropathiques post-chimiothérapiques.

Aussi le problème que se propose de résoudre l'invention est de trouver un nouveau traitement de la douleur provoquée par un traitement par un agent anti-cancéreux.

De manière inattendue, les inventeurs ont mis en évidence que l'administration de neurotoxine botulique présente un effet analgésique dans le traitement de la douleur provoquée par la chimiothérapie.

Dans ce but la présente invention propose l'utilisation d'au moins une neurotoxine botulique pour l'obtention d'un médicament destiné à traiter ou prévenir la ou les douleurs post-chimiothérapiques.

L'invention offre des avantages déterminants, en particulier celui d'éviter ou de prévenir la douleur suite à un traitement par un agent anti-cancéreux et ainsi permettre l'augmentation des doses de traitement sans augmenter la douleur.

Enfin l'invention a pour avantage de pouvoir être mise en oeuvre dans toutes industries, notamment l'industrie pharmaceutique, vétérinaire, cosmétique.

D'autres avantages et caractéristiques de l'invention apparaîtront clairement à la lecture de la description et des exemples donnés à titre purement illustratifs et non limitatifs qui vont suivre.

Par « douleur » au sens de la présente invention, il faut entendre « toute expérience désagréable émotionnelle et sensorielle associée à un dommage tissulaire présent ou potentiel, ou décrite par le patient en de tels termes ».

Par l'expression neurotoxine botulique, on entend une toxine botulique qui est soit une protéine libre (i.e. libre de toute protéine la complexant), soit un complexe protéique, ledit complexe protéique pouvant comprendre par exemple de l'hémagglutinine (protéine HA) associée à de la toxine botulique, ou soit un fragment protéique.

Par l'expression toxine botulique, on entend une molécule possédant l'activité biologique de la toxine botulique, qui peut être par exemple soit une protéine, soit un polypeptide, soit un peptide, soit une protéine de fusion, soit une protéine tronquée, soit une protéine chimérique, soit une protéine mutée ou une protéine recombinante.

Par l'expression activité biologique de la toxine botulique, on entend au sens de la présente invention soit une paralysie musculaire soit une inhibition de l'exocytose, en particulier de l'exocytose de l'acétylcholine ou d'un autre neurotransmetteur.

Par protéine, polypeptide ou peptide on entend au sens de la présente invention, un polymère d'acides aminés, naturels ou non, lévogyres ou non, dextrogyres ou non.

Par protéine chimérique, on entend au sens de la présente invention une protéine obtenue après association de différents types de molécules, par exemple après association de lipides, de glycolipides, de peptides, de polypeptides, de protéines, de glycoprotéines, de carbohydrates, de polysaccharides, d'acides nucléiques, de polyéthylène glycol, etc.

La toxine botulique, en particulier la toxine botulique de type A1 (Dysport^{®} commercialisé par Ipsen ou Botox^{®} commercialisé par Allergan), est utilisée depuis les années 80 chez l'homme pour le traitement de maladies / désordres divers et variés. Parmi les maladies / désordres pouvant être traités par la toxine botulique, on peut citer entre autres des désordres musculaires (par exemple le blépharospasme, la spasticité de l'adulte ou de l'enfant ou encore le torticolis), la migraine, la douleur d'origine musculaire, le diabète, l'hyperhydrose (ou transpiration excessive), l'hypersalivation ou même les rides.

WO 01/78760A décrit un traitement de douleurs par l'administration périphérique de la toxine botulinique, y compris des douleurs dû à une blessure, une intervention chirurgicale, une infection, un accident ou une maladie, y compris le cancer et le diabète, y compris les maladies et les troubles neuropathiques, où la douleur n'est pas principalement due à un spasme ou une condition hypertonique musculaire.

La neurotoxine botulique, pure ou quasiment pure, peut être obtenue à partir d'un complexe protéique comprenant de la toxine botulique par exemple selon la méthode décrite dans Current topics in Microbiology and Immunology (1995), 195, p. 151-154. Une neurotoxine botulique, pure ou quasiment pure, peut être obtenue par exemple, par purification d'un milieu de fermentation ou bouillon de culture contenant une souche de *Clostridium Botulinum,* et enrichi par exemple en viande ou en nourriture protéinée.

La présente invention a tout d'abord pour objet l'utilisation d'au moins une neurotoxine botulique pour l'obtention d'un médicament destiné à traiter ou prévenir la ou les douleurs post-chimiothérapiques par administration localisée par voie intramusculaire, intra dermique ou sous-cutanée permettant d'obtenir un effet généralisé, la ou les douleurs n'étant pas de douleur(s) induite(s) par des tumeurs.

De préférence, la présente invention n'a pas pour objet de traiter les cancers, ni les tumeurs, ni les douleurs induites par des tumeurs, en particulier les douleurs associées aux tumeurs osseuses.

De préférence, l'utilisation selon l'invention d'au moins une neurotoxine botulique pour l'obtention d'un médicament permet de traiter ou de prévenir les douleurs post-chimiothérapiques induites par un agent anti-cancéreux ou ses sels ou ses dérivés.

De préférence, l'agent anti-cancéreux ou ses sels ou ses dérivés sont choisis parmi les composés suivants ou leurs mélanges : les taxanes, les sels de platine, ou d'autres agents anti-cancéreux.

De préférence, l'agent anti-cancéreux ou ses sels ou ses dérivés sont choisis parmi les taxanes comme par exemple le docetaxel, le paclitaxel (taxol) ou leurs mélanges.

De préférence, l'agent anti-cancéreux ou ses sels ou ses dérivés sont choisis parmi les sels de platine comme par exemple le cisplatine, l'oxaliplatine ou le carboplatine ou leurs mélanges.

De préférence, l'agent anti-cancéreux ou ses sels ou ses dérivés sont choisis parmi la vincristine, la vinblastine, l'étoposide, la teniposide, l'Ara-A (adenoside-arabinoside), l'Ara-C (cytarabine), le fluorouracil, la procarbazine, la vinorelbine, la gemcitabine, ou encore des produits ou mélanges de produits comme paclitaxel / carboplatine, paclitaxel / anthracyclines, paclitaxel / carboplatine / gemcitabine, paclitaxel / estramustine, docetaxel / cisplatine, docetaxel / doxorubicine, docetaxel / vinorelbine, docetaxel / trastuzumab, docetaxel / capecitabine ou cisplatine / cyclophosphamide, cisplatine / irinotecan, carboplatine / topotecan, carboplatine / estramustine, etoposide / estramustine, vinblastine / estramustine.

Par sel, on entend un sel pharmaceutiquement acceptable et notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", Int. J. Pharm. (1986), 33, 201-217.

De manière préférentielle, la neurotoxine botulique permet d'obtenir un effet systémique.

Par « effet systémique », on entend au sens de la présente invention une administration localisée permettant d'obtenir un effet généralisé.

Selon une utilisation préférée de l'invention la neurotoxine botulique est administrée par voie intramusculaire, intra-dermique ou sous-cutanée.

De préférence la neurotoxine botulique utilisée selon l'invention est choisie parmi les neurotoxines botuliques de type A, A1, A2, B, C, C1, D, E, F ou G.

La neurotoxine botulique de type A1 correspond en fait à la toxine botulique classique qui est communément appelée toxine botulique de type A, sans distinction du sous-type. La neurotoxine botulique de type A1 est commercialisée sous le nom de DYSPORT^{®} ou BOTOX^{®}.

Selon l'invention, la neurotoxine botulique de type A1 peut correspondre soit à un complexe de toxine botulique A1 et d'hémagglutinine, soit à la toxine botulique de type A1 libre de toutes protéines complexantes.

La toxine botulique de type A2 a d'abord été isolée à partir de cas d'enfants atteints de botulisme vers 1990 (Sakaguchi et al., Int. J. Food Microbiol. (1990), 11, 231-242). Cette toxine est immunologiquement et biochimiquement différente de la toxine botulique de type A1.

La toxine botulique de type A2 peut être isolée à partir des souches suivantes : Kyoto-F, Chiba-H, Y-8036, 7103-H, 7105-H, KZ1828, NCTC2012 ou NCTC9837 (Cordoba et al., System. Appl. Microbiol. (1995), 18, 13-22; Franciosa et al., abstract presented at 40th Interagency Botulism Research Coordinating Committee (IBRCC) Meeting, November 2003).

De préférence la neurotoxine botulique utilisée selon l'invention est la toxine botulique de type A1.

Selon une variante de l'invention, la neurotoxine botulique utilisée selon l'invention est la toxine botulique de type A2 isolée à partir de la souche *Clostridium botulinum* référencée et accessible sous le numéro NCTC9837, auprès de la National Collection of Type Cultures - Central Public Health Laboratory - London - UK. La souche NCTC9837 est parfois appelée souche Mauritius 1955.

La toxine botulique de type A2 diffère de la toxine A1 par, entre autre, sa séquence en acides aminés, son poids moléculaire, ses caractéristiques immunologiques et génétiques (Kubota et al., Biochem. Biophys. Res. Commun. (1996), 224 (3), 843-848).

Selon un mode préférentiel, la neurotoxine botulique utilisée selon l'invention est une neurotoxine botulique modifiée ayant au moins un acide aminé supprimé, modifié ou remplacé.

De préférence la neurotoxine botulique utilisée selon l'invention est associée à au moins un polysaccharide ou un mélange de plusieurs polysaccharides.

Par polysaccharide, on entend au sens de la présente invention, un polymère comprenant au moins 2 monomères, les monomères étant des saccharides. Cette définition inclut les disaccharides.

Dans le cadre de l'invention, les polysaccharides peuvent être ioniques et/ou non ioniques.

De préférence, la composition comprend au moins un polysaccharide comprenant majoritairement des unités de glucose. Le terme majoritairement signifiant que le glucose est majoritaire en nombre d'unités de monomère.

Comme exemple de polysaccharides convenant selon l'utilisation de l'invention, on peut citer l'amidon, les dérivés d'amidon, l'hydroxyethyle amidon en particulier le 2-hydroxy-ethyl amidon.

Les polysaccharides convenant selon la présente invention peuvent être substitués, en particulier peuvent être substitués par des radicaux alkyles, alcoxy, ou encore par des radicaux alkyles eux-mêmes substitués par des fonctions alcools.

Selon une variante de l'invention, la quantité de polysaccharide convenant selon la présente invention est d'au moins 1 µg de polysaccharide pour 1 unité de toxine botulique. Selon le choix du polysaccharide, il est possible d'utiliser au moins 0,5 µg de polysaccharide pour 1 unité de toxine botulique.

De préférence la neurotoxine botulique utilisée selon l'invention est associée à au moins un tensio-actif ou un mélange de plusieurs tensio-actifs.

Par agent tensio-actif, on entend au sens de l'invention un agent émulsifiant ou un agent solubilisant.

Dans le cadre de l'invention les tensio-actifs mis en oeuvre peuvent être choisis parmi les tensio-actifs cationiques, anioniques ou non-ioniques.

De préférence la neurotoxine botulique utilisée selon l'invention est associée à au moins un tensio-actif ou un mélange de plusieurs tensio-actifs, choisi parmi les tensio-actifs cationiques, anioniques ou non-ioniques.

De préférence la neurotoxine botulique utilisée selon l'invention est associée à au moins un tensio-actif choisi parmi les tensio-actifs non-ioniques du groupe des polysorbates.

Parmi le groupe des polysorbates, on peut citer le polysorbate 20, le polysorbate 21, le polysorbate 40, le polysorbate 60, le polysorbate 61, le polysorbate 65, le polysorbate 80, le polysorbate 81, le polysorbate 85, le polysorbate 120, le polysorbate 80 acetate.

Le tensio-actif préféré selon une variante de l'invention est le polysorbate 80.

La neurotoxine botulique utilisée selon l'invention peut être administrée de préférence par injection comme par exemple par injection intramusculaire, intra-dermique ou sous-cutanée, ou encore par application topique par exemple application d'un patch.

Dans le cas des injections selon l'invention, la neurotoxine botulique pourra être associée à un agent facilitant l'injection encore appelé véhicule d'injection ou vecteur d'injection.

La dose d'utilisation selon la présente invention de la neurotoxine botulique, à prévoir pour le traitement ou la prévention des maladies ou troubles mentionnés ci-dessus, varie suivant le mode d'administration, l'âge et le poids corporel du sujet à traiter ainsi que l'état de ce dernier, et il en sera décidé en définitive par le médecin ou le vétérinaire traitant. Une telle quantité déterminée par le médecin ou le vétérinaire traitant est appelée ici "quantité thérapeutiquement efficace".

De préférence, la neurotoxine botulique utilisée selon l'invention est administrée à une dose comprise entre 0,01 U et 1500 U, préférentiellement à une dose comprise entre 0,01 U et 1000 U, plus préférentiellement de 0,1 à 500 U, plus particulièrement à une dose comprise entre 0,1 et 100 U, encore plus particulièrement à une dose comprise entre 1 et 20 U, et ceci quel que soit le type de toxine botulique ou quelle que soit sa provenance. (L'unité de toxine (U) est définie dans la partie expérimentale).

La présente invention a pour objet l'utilisation de la neurotoxine botulique, décrite ci-dessus, pour l'obtention d'un médicament destiné à traiter ou prévenir la ou les douleurs post-chimiothérapiques, c'est-à-dire les douleurs liées à un traitement contre le cancer.

Par l'expression « cancer », on entend au sens de l'invention tout type de cancer, c'est dire invasif, non invasif, infiltrant, hormonal, non hormonal, localisé ou métastatique.

Selon une utilisation préférée de l'invention la neurotoxine botulique permet de traiter ou prévenir la ou les douleurs post-chimiothérapiques chez des patients atteints par exemple de cancers du colon, du rectum, du sein, des poumons, du pancréas, des testicules, du rein, de l'utérus, de l'ovaire, de la prostate, de la peau, des os, de la moelle épinière ainsi que les patients atteints de sarcomes, de carcinomes, de fibroadénomes, de neuroblastomes, de leucémies, de lymphomes, ou de mélanomes.
La figure 1 présente l'effet de la toxine botulique de type A1 sur la patte droite suite à l'injection par voie sous-plantaire dans la patte droite (ipsilatérale) dans le modèle de neuropathie périphérique induite par le paclitaxel.
La figure 2 présente l'effet de la toxine botulique de type A1 sur la patte gauche (contralatérale) suite à l'injection par voie sous-plantaire dans la patte droite (ipsilatérale) dans le modèle de neuropathie périphérique induite par le paclitaxel.

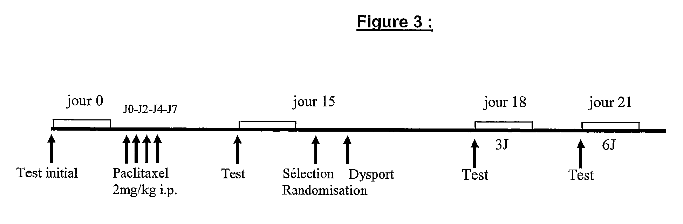

La figure 3 présente le protocole d'injection.

Les exemples suivants illustrent l'invention sans en limiter la portée.

### EXEMPLES

La mesure de la quantité des neurotoxines botuliques utilisées selon l'invention a été réalisée par la mesure d'une dose létale DL₅₀. Par DL₅₀, on entend au sens de la présente invention la dose létale ou encore dose semi létale d'une substance donnée. Il s'agit de la dose (ou quantité) qui conduit à la mort de 50% des animaux testés d'un groupe. Une unité de toxine (U) correspond à la DL₅₀ chez la souris par voie intra péritonéale.

### Modèle de neuropathie induite par administration d'un agent anti-cancéreux :

L'activité du Dysport^{®} (toxine botulique de type A1) a été évaluée in vivo sur un modèle de neuropathie périphérique induite par administration d'un agent anti-cancéreux : le Paclitaxel (Taxol^{®}).

Des rats mâles Sprague Dawley (Charles River) de 160g environ sont laissés en stabulation pendant 6 jours dans les conditions d'animalerie. 4 groupes sont constitués d'au moins 10 animaux.

La neuropathie est induite par injections intra-péritonéales (i.p.) de 2mg/kg de paclitaxel aux jours J0, J2, J4 et J7.

Avant la première injection, les rats sont numérotés et pesés et la nociception (seuil de la douleur) est évaluée après un stimulus mécanique dont la pression est croissante : induction d'une pression initiale (210g/mm²) sur les deux pattes arrières des rats effectuée à l'aide d'un analgésie-mètre selon la méthode de Randall et Selitto. Ces mesures permettent de définir les valeurs basales avant le développement de la neuropathie (J0).

La diminution du seuil nociceptif correspondant à l'atteinte neuropathique est maximale entre le 14^{éme} et le 24^{éme} jour après la première injection de paclitaxel. Le seuil de nociception des deux pattes arrières des rats est diminué semblablement. Les études de neuropathie seront donc effectuées entre le 14^{éme} et le 24^{éme} jour sur les 2 pattes arrières des rats. Dans le cas décrit, les mesures de nociception sont effectuées à J18 et J21.

Le jour de l'expérience (J15), les rats sont pesés, la nociception est mesurée et les animaux n'ayant pas développé la neuropathie ce jour (diminution de la nociception par rapport aux mesures à J0) sont exclus de l'étude. Le Dysport est injecté en sous-plantaire dans la patte arrière droite (ipsilatérale) des rats et la nociception est mesurée sur les 2 pattes arrières (ipsilatérale et contralatérale) 3 jours et 6 jours après son administration.

### Effet du Dysport sur la neuropathie induite par le taxol :

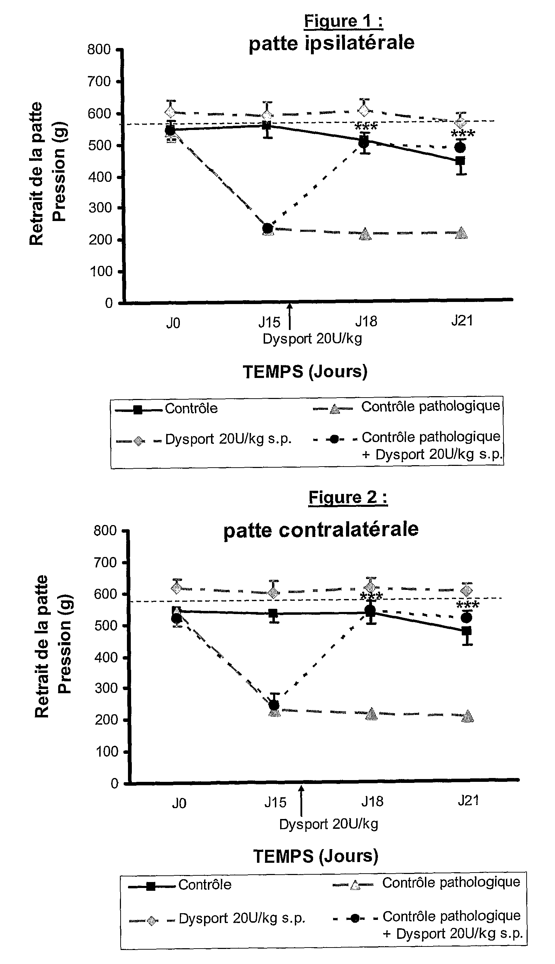

La figure 1 représente l'effet du Dysport (de la toxine botulique de type A1) sur la patte droite suite à son injection par voie sous-plantaire dans la patte droite (ipsilatérale) dans le modèle de neuropathie périphérique induite par le paclitaxel.

Le contrôle indique le seuil de douleur toléré par le rat lorsque l'on applique une pression croissante sur ses pattes ; ce groupe a été traité par le véhicule du paclitaxel (montanox 3% dans NaCl 0,9%) par voie intra-péritonéale et par le véhicule du Dysport (NaCl 0,9%) en sous-plantaire. De J0 à J21, le seuil de nociception se situe vers 500 g/mm².

Le contrôle pathologique indique le seuil de douleur toléré par le rat lorsque l'on applique une pression croissante sur ses pattes ; ce groupe a été traité par le paclitaxel par voie intra-péritonéale et par le véhicule du Dysport (NaCl 0,9%) en sous-plantaire. Au jour zéro ce seuil se situe à 544 g/mm² puis diminue pour atteindre 232 g/mm² au jour 15, 216 g/mm² au jour 18 et 216 g/mm² au jour 21. Ces résultats indiquent qu'après injection i.p. de paclitaxel, la sensibilité des pattes des rats est augmentée suite à l'application d'une pression sur celles-ci.

L'administration du Dysport à la dose de 20U/kg en sous-plantaire dans un groupe traité uniquement par le véhicule du paclitaxel (montanox 3% dans NaCl 0,9%) par voie intra-péritonéale, indique que le seuil de douleur toléré par le rat n'est pas significativement modifié. Le seuil de douleur suite à un stimulus mécanique appliqué sur les pattes des rats se situe de J0 à J21 vers 590 g/ mm².

L'administration du Dysport à la dose de 20U/kg s.p. dans un groupe traité par le paclitaxel indique que le seuil de douleur toléré par le rat sur sa patte droite augmente. Le seuil de douleur suite à un stimulus mécanique appliqué sur les pattes des rats, est significativement augmenté pour atteindre 500 g/mm² à J18 (soit 3 jours après le traitement avec le Dysport) et 480 g/mm² à J21 (soit 6 jours après le traitement avec le Dysport) contre 232 g/mm² à J15 (avant Dysport).

La figure 2 présente l'effet du Dysport (toxine botulique de type A1) sur la patte gauche (contralatérale) suite à l'injection par voie sous-plantaire dans la patte droite (ipsilatérale) dans le modèle de neuropathie périphérique induite par le paclitaxel.

Le contrôle indique le seuil de douleur toléré par le rat lorsque l'on applique une pression croissante sur ses pattes ; ce groupe a été traité par le véhicule du paclitaxel (montanox 3% dans NaCl 0,9%) par voie intra-péritonéale et par le véhicule du Dysport (NaCl 0,9%) en sous-plantaire. De J0 à J21, le seuil de nociception se situe vers 500 g/mm².

Le contrôle pathologique indique le seuil de douleur toléré par le rat lorsque l'on applique une pression croissante sur ses pattes ; ce groupe a été traité par le paclitaxel par voie intra-péritonéale et par le véhicule du Dysport (NaCl 0,9%) en sous-plantaire. Au jour zéro ce seuil se situe à 536 g/mm² puis diminue pour atteindre 228 g/mm² au jour 15, 216 g/mm² au jour 18 et 204 g/mm² au jour 21. Ces résultats indiquent qu'après injection i.p. de paclitaxel, la sensibilité des pattes des rats est augmentée suite à l'application d'une pression sur celles-ci.

L'administration du Dysport à la dose de 20U/kg en sous-plantaire dans un groupe traité uniquement par le véhicule du paclitaxel (montanox 3% dans NaCl 0,9%) par voie intra-péritonéale, indique que le seuil de douleur toléré par le rat n'est pas significativement modifié. Le seuil de douleur suite à un stimulus mécanique appliqué sur les pattes des rats se situe de J0 à J21 vers 610 g/mm².

L'administration dans la patte droite du Dysport à la dose de 20U/kg s.p. dans un groupe traité par le paclitaxel indique que le seuil de douleur toléré par le rat sur sa patte gauche augmente. Le seuil de douleur suite à un stimulus mécanique appliqué sur les pattes des rats, est significativement augmenté pour atteindre 540 g/mm² à J18 (soit 3 jours après le traitement avec le Dysport) et 512 g/mm² à J21 (soit 6 jours après le traitement avec le Dysport) contre 244 g/mm² à J15 (avant Dysport).

Ces résultats indiquent que l'administration de Dysport en sous-plantaire dans la patte droite des rats induit un effet analgésique, mesuré sur les deux pattes arrières des rats, dans ce test de neuropathie périphérique induite par quatre injections consécutives et systémiques de paclitaxel.

## Revendications

1. Utilisation d'au moins une neurotoxine botulique pour l'obtention d'un médicament destiné à traiter ou prévenir la ou les douleurs post-chimiothérapiques par administration localisée par voie intramusculaire, intra dermique ou sous-cutanée permettant d'obtenir un effet généralisé, la ou les douleurs n'étant pas de douleur(s) induite(s) par des tumeurs.

2. Utilisation selon la revendication 1 **caractérisé en ce que** la ou les douleurs post-chimiothérapiques sont induites par un agent anti-cancéreux ou ses sels.

3. Utilisation selon l'une des revendications précédentes **caractérisée en ce que** la ou les douleurs post-chimiothérapiques sont provoquées par un agent anti-cancéreux ou ses sels choisis parmi les composés suivants ou leurs mélanges : les taxanes ou les sels de platine.

4. Utilisation selon l'une des revendications précédentes **caractérisée en ce que** la ou les douleurs post-chimiothérapiques sont provoquées par un agent anti-cancéreux ou ses sels choisis parmi le docetaxel, le paclitaxel (taxol) ou leurs mélanges.

5. Utilisation selon la revendication 3 **caractérisée en ce que** la ou les douleurs post-chimiothérapiques sont provoquées par un agent anti-cancéreux ou ses sels choisis parmi le cisplatine, l'oxaliplatine ou le carboplatine ou leurs mélanges.

6. Utilisation selon l'une des revendications précédentes **caractérisée en ce que** la ou les douleurs post-chimiothérapiques sont provoquées par un agent anti-cancéreux ou ses sels choisis parmi la vincristine, la vinblastine, l'étoposide, la teniposide, l'Ara-A (adenoside-arabinoside), l'Ara-C (cytarabine), le fluorouracil, la procarbazine, la vinorelbine, la gemcitabine, ou encore des produits ou mélanges de produits comme paclitaxel/ carboplatine, paclitaxel / anthracyclines, paclitaxel / carboplatine/gemcitabine, paclitaxel / estramustine, docetaxel / cisplatine, docetaxel / doxorubicine, docetaxel / vinorelbine, docetaxel / trastuzumab, docetaxel / capecitabine ou cisplatine / cyclophosphamide, cisplatine / irinotecan, carboplatine / topotecan, carboplatine / estramustine, etoposide / estramustine, vinblastine / estramustine.

7. Utilisation selon l'une des revendications précédentes **caractérisée en ce que** la neurotoxine botulique est choisie parmi les neurotoxines botuliques de type A, A1, A2, B, C, C1, D, E, F ou G.

8. Utilisation selon l'une des revendications précédentes **caractérisée en ce que** la neurotoxine botulique est la toxine botulique de type A1.

9. Utilisation selon l'une des revendications précédentes **caractérisée en ce que** la neurotoxine botulique est associée à au moins un polysaccharide ou un mélange de plusieurs polysaccharides.

10. Utilisation selon la revendication 9 **caractérisée en ce que** le polysaccharide est le 2-hydroxy-ethyl amidon.

11. Utilisation selon l'une des revendications précédentes **caractérisée en ce que** la neurotoxine botulique est une neurotoxine botulique modifiée ayant au moins un acide aminé supprimé, modifié ou remplacé.

12. Utilisation selon l'une des revendications précédentes **caractérisée en ce que** la neurotoxine botulique est associée à au moins un tensio-actif ou un mélange de plusieurs tensio-actifs, choisis parmi les tensio-actifs cationiques, anioniques ou non-ioniques.

13. Utilisation selon l'une des revendications précédentes **caractérisée en ce que** la neurotoxine botulique est associée à au moins un tensio-actif choisi parmi les tensio-actifs non-ioniques du groupe des polysorbates.

14. Utilisation selon l'une des revendications précédentes **caractérisée en ce que** la neurotoxine botulique est administrée à une dose comprise entre 0,01 U et 1500 U.

15. Utilisation selon l'une des revendications précédentes **caractérisée en ce que** la ou les douleurs post-chimiothérapiques provient de patients atteints de cancers du colon, du rectum, du sein, des poumons, du pancréas, des testicules, du rein, de l'utérus, de l'ovaire, de la prostate, de la peau, des os, de la moelle épinière ainsi que les patients atteints de sarcomes, de carcinomes, de fibroadénomes, de neuroblastomes, de leucémies, de lymphomes, ou de mélanomes.

## Patentansprüche

1. Verwendung wenigstens eines Botulinum-Neurotoxins zur Herstellung eines Medikaments, das dazu bestimmt ist, den Schmerz oder die Schmerzen nach Chemotherapie durch lokalisierte Verabreichung auf intramuskulärem, intradermalem oder subkutanem Weg, die ermöglicht, eine generalisierte Wirkung zu erzielen, zu behandeln oder zu verhindern, wobei der Schmerz oder die Schmerzen kein Schmerz oder keine Schmerzen, der/die durch Tumore induziert ist/sind, ist/sind.

2. Verwendung nach Anspruch 1, **dadurch gekennzeichnet, dass** der Schmerz oder die Schmerzen nach Chemotherapie durch ein Antikrebsmittel oder seine Salze induziert ist/sind.

3. Verwendung nach einem der vorangehenden Ansprüche, **dadurch gekennzeichnet, dass** der Schmerz oder die Schmerzen nach Chemotherapie hervorgerufen wird/werden durch ein Antikrebsmittel oder seine Salze, ausgewählt aus den folgenden Verbindungen oder ihren Gemischen:
Taxane oder Platinsalze.

4. Verwendung nach einem der vorangehenden Ansprüche, **dadurch gekennzeichnet, dass** der Schmerz oder die Schmerzen nach Chemotherapie durch ein Antikrebsmittel oder seine Salze, ausgewählt aus Docetaxel, Paclitaxel (Taxol) und ihren Gemischen, hervorgerufen wird/werden.

5. Verwendung nach Anspruch 3, **dadurch gekennzeichnet, dass** der Schmerz oder die Schmerzen nach Chemotherapie durch ein Antikrebsmittel oder seine Salze, ausgewählt aus Cisplatin, Oxaliplatin oder Carboplatin oder ihren Gemischen, hervorgerufen wird/werden.

6. Verwendung nach einem der vorangehenden Ansprüche, **dadurch gekennzeichnet, dass** der Schmerz oder die Schmerzen nach Chemotherapie durch ein Antikrebsmittel oder seine Salze, ausgewählt aus Vincristin, Vinblastin, Etoposid, Teniposid, Ara-A (Adenosidarabinosid), Ara-C (Cytarabin), Fluoruracil, Procarbazin, Vinorelbin, Gemcitabin und auch den Produkten oder Gemischen von Produkten, wie Paclitaxel / Carboplatin, Paclitaxel / Anthracycline, Paclitaxel / Carboplatin / Gemcitabin, Pactitaxel / Estramustin, Docetaxel / Cisplatin, Docetaxel / Doxorubicin, Docetaxel / Vinorelbin, Docetaxel / Trastuzumab, Docetaxel / Capecitabin oder Cisplatin / Cyclophosphamid, Cisplatin / Irinotecan, Carboplatin / Topotecan, Carboplatin / Estramustin, Etoposid / Estramustin, Vinblastin / Estramustin, hervorgerufen wird/werden.

7. Verwendung nach einem der vorangehenden Ansprüche, **dadurch gekennzeichnet, dass** das Botulinum-Neurotoxin aus den Botulinum-Neurotoxinen des Typs A, A1, A2, B, C, C1, D, E, F oder G ausgewählt ist.

8. Verwendung nach einem der vorangehenden Ansprüche, **dadurch gekennzeichnet, dass** das Botulinum-Neurotoxin das Botulinum-Neurotoxin des Typs A1 ist.

9. Verwendung nach einem der vorangehenden Ansprüche, **dadurch gekennzeichnet, dass** das Botulinum-Neurotoxin mit wenigstens einem Polysaccharid oder einem Gemisch mehrerer Polysaccharide assoziiert ist.

10. Verwendung nach Anspruch 9, **dadurch gekennzeichnet, dass** das Polysaccharid 2-Hydroxyethylstärke ist.

11. Verwendung nach einem der vorangehenden Ansprüche, **dadurch gekennzeichnet, dass** das Botulinum-Neurotoxin ein modifiziertes Botulinum-Neurotoxin ist, das wenigstens eine Aminosäure supprimiert, modifiziert oder ersetzt hat.

12. Verwendung nach einem der vorangehenden Ansprüche, **dadurch gekennzeichnet, dass** das Botulinum-Neurotoxin mit wenigstens einem Surfaktant oder einem Gemisch mehrerer Surfaktanten, ausgewählt aus kationischen, anionischen oder nicht-ionischen Surfaktanten, assoziiert ist.

13. Verwendung nach einem der vorangehenden Ansprüche, **dadurch gekennzeichnet, dass** das Botulinum-Neurotoxin mit wenigstens einem Surfaktant, ausgewählt aus nicht-ionischen Surfaktanten der Gruppe der Polysorbate, assoziiert ist.

14. Verwendung nach einem der vorangehenden Ansprüche, **dadurch gekennzeichnet, dass** das Botulinum-Neurotoxin in einer Dosis zwischen 0,01 U und 1500 U verabreicht wird.

15. Verwendung nach einem der vorangehenden Ansprüche, **dadurch gekennzeichnet, dass** der Schmerz oder die Schmerzen nach Chemotherapie bei Patienten auftritt/auftreten, die an Krebs des Kolons, des Rektums, der Brust, der Lungen, des Pankreas, der Hoden, der Niere, des Uterus, des Eierstocks, der Prostata, der Haut, der Knochen, des Rückenmarks erkrankt sind, sowie bei Patienten, die an Sarkomen, Karzinomen, Fibroadenomen, Neuroplastomen, Leukämien, Lymphomen oder Melanomen erkrankt sind.

## Claims

1. Use of at least one botulinum neurotoxin for obtaining a medicament intended to treat or prevent post-chemotherapy pain or pains by local administration by an intramuscular, intradermal, or subcutaneous route allowing to obtain a generalized effect, said pains or pains are not pain(s) induced by tumors.

2. Use according to claim 1, wherein said post-chemotherapy pain or pains are induced by an anti-neoplastic agent or its salts.

3. Use according to any one of the preceding claim, wherein the post-chemotherapy pain or pains are caused by an anti-neoplastic agent or its salts chosen from the following compounds or their mixtures: taxanes or platinum salts.

4. Use according to any one of the preceding claims, wherein the post-chemotherapy pain or pains are caused by an anti-neoplastic agent or its salts chosen from docetaxel, paclitaxel (taxol) or their mixtures.

5. Use according to claim 3, wherein the post-chemotherapy pain or pains are caused by an anti-neoplastic agent or its salts chosen from cisplatin, oxaliplatin or carboplatin or their mixtures.

6. Use according to any one of the preceding claims, wherein the post-chemotherapy pain or pains are caused by an anti-neoplastic agent or its salts chosen from vincristine, vinblastine, etoposide, teniposide, Ara-A (adenoside-arabinoside), Ara-C (cytarabine), fluorouracil, procarbazine, vinorelbine, gemcitabine, or further products or mixtures of products such as paclitaxel / carboplatin, paclitaxel / anthracyclines, paclitaxel / carboplatin / gemcitabine, paclitaxel / estramustine, docetaxel / cisplatin, docetaxel / doxorubicin, docetaxel / vinorelbine, docetaxel / trastuzumab, docetaxel / capecitabine or cisplatin / cyclophosphamide, cisplatin / irinotecan, carboplatin / topotecan, carboplatin / estramustine, etoposide / estramustine, vinblastine / estramustine.

7. Use according to any one of the preceding claims, wherein the botulinum neurotoxin is chosen from the botulinum neurotoxins of type A, A1, A2, B, C, C1, D, E, F or G.

8. Use according to any one of the preceding claims, wherein the botulinum neurotoxin is botulinum toxin type A1.

9. Use according to any one of the preceding claims, wherein the botulinum neurotoxin is combined with at least one polysaccharide or a mixture of several polysaccharides.

10. Use according to claim 9, wherein the polysaccharide is 2-hydroxy-ethyl starch.

11. Use according to any one of the preceding claims, wherein the botulinum neurotoxin is a modified botulinum neurotoxin having at least one deleted, modified or replaced amino acid.

12. Use according to any one of the preceding claims, wherein the botulinum neurotoxin is combined with at least one surfactant or a mixture of several surfactants, chosen from the cationic, anionic or non-ionic surfactants.

13. Use according to any one of the preceding claims, wherein the botulinum neurotoxin is combined with at least one surfactant chosen from the non-ionic surfactants of the polysorbates group.

14. Use according to any one of the preceding claims, wherein the botulinum neurotoxin is administered at a dose comprised between 0.01 U and 1500 U.

15. Use according to any one of the preceding claims, wherein the post-chemotherapy pain or pains affect patients suffering from cancers of the colon, rectum, breast, lungs, pancreas, testicles, kidney, uterus, ovary, prostate, skin, bones, spinal cord as well as patients suffering from sarcomas, carcinomas, fibroadenomas, neuroblastomas, leukaemias, lymphomas, or melanomas.
